(19) Europäisches Patentamt
European Patent Office
Office européen des brevets

(11) **EP 3 621 513 B1**

(12) **EUROPÄISCHE PATENTSCHRIFT**

(45) Veröffentlichungstag und Bekanntmachung des Hinweises auf die Patenterteilung:
**14.01.2026   Patentblatt 2026/03**

(21) Anmeldenummer: **18723835.7**

(22) Anmeldetag: **11.05.2018**

(51) Internationale Patentklassifikation (IPC):
**A61B 5/03** (2006.01)      **A61B 5/0205** (2006.01)
**A61B 5/00** (2006.01)      **A61B 8/08** (2006.01)
**A61B 8/00** (2006.01)      A61B 5/024 (2006.01)
A61B 5/053 (2021.01)      A61B 5/08 (2006.01)

(52) Gemeinsame Patentklassifikation (CPC):
**A61B 5/031; A61B 5/0205; A61B 5/7235;**
**A61B 8/0808; A61B 8/485;** A61B 5/024;
A61B 5/053; A61B 5/0816

(86) Internationale Anmeldenummer:
**PCT/EP2018/062262**

(87) Internationale Veröffentlichungsnummer:
**WO 2018/206799 (15.11.2018 Gazette 2018/46)**

(54) **EINRICHTUNG ZUR BESTIMMUNG EINER KENNGRÖSSE ZUR DIAGNOSE DES HYDROZEPHALUS UND ANDERER STÖRUNGEN DES HIRNDRUCKS**

DEVICE FOR DETERMINING A CHARACTERISTIC FOR DIAGNOSING HYDROCEPHALUS AND OTHER DISORDERS OF THE BRAIN PRESSURE

DISPOSITIF POUR DÉTERMINER UN PARAMÈTRE PERMETTANT LE DIAGNOSTIC DE L'HYDROCÉPHALIE ET D'AUTRES TROUBLES DE LA PRESSION INTRA-CRÂNIENNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.05.2017   DE 102017004576**
**23.09.2017   EP 17192789**
**12.01.2018   DE 102018100697**

(43) Veröffentlichungstag der Anmeldung:
**18.03.2020   Patentblatt 2020/12**

(73) Patentinhaber:
• **Spiegelberg, Andreas**
**8810 Horgen (CH)**
• **Universität Zürich**
**8006 Zürich (CH)**

(72) Erfinder:
• **SPIEGELBERG, Andreas**
**8810 Horgen (CH)**
• **KURTCUOGLU, Vartan**
**8409 Winterthur (CH)**

(74) Vertreter: **Schulz Junghans**
**Patentanwälte PartGmbB**
**Großbeerenstraße 71**
**10963 Berlin (DE)**

(56) Entgegenhaltungen:
US-A1- 2006 079 773      US-A1- 2013 109 979
US-B1- 6 387 051

• JETZKI S ET AL: "Analysis of Pulse Waves in Intracranial Pressure", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCI�T� FRAN�AISE DE G�NIE BIOLOGIQUE ET M�DICAL (SFGB, 22 August 2007 (2007-08-22), pages 2863 - 2866, XP031336805, ISBN: 978-1-4244-0787-3

# EP 3 621 513 B1

**Beschreibung**

Die vorliegende Erfindung betrifft eine Einrichtung zur Bestimmung einer Kenngrösse zur Diagnose des Hydrozephalus und anderer Störungen des Hirndrucks sowie ein Verfahren zur Bestimmung einer Kenngröße des Kopfinhaltes von Menschen und anderen Säugetieren.

Für die Diagnostik von Patienten, bei denen ein Ungleichgewicht zwischen der Bildung von Hirnwasser und der Rückresorption von Hirnwasser besteht, den sogenannten Hydrozephaluspatienten, und auch bei anderen Patienten, bei denen die Druckverhältnisse im Kopf bzw. im Gehirn gestört sind, werden unter anderem Einrichtungen verwandt, welche den Druck oder die Volumenelastizität des Raumes im Schädel bestimmen. Bei vielen dieser Patienten ist der mittlere Druck im Hirnwasser nicht pathologisch erhöht. Die Volumen-elastizität des Inhalts des Schädels ist jedoch eingeschränkt und es kommt bei kleinsten auslösenden Druckänderungen zu wellenförmigen Erhöhungen des Druckes, insbesondere im Schlaf und im Liegen. Bei anderen dieser Patienten geht die Minderung der Volumenelastizität auch mit einer Erhöhung des Druckes einher.

Die Volumenelastizität ist eine Kenngrösse für das Vermögen des Gehirns, Volumenänderungen zu kompensieren. Das Gehirn ist in dem starren knöchernen Schädel eingeschlossen. Neben der Hirnsubstanz sind in diesem Hohlraum Arterien, die das Blut zum Gehirn zuführen, Venen, die das Blut vom Gehirn wegführen, und Liquorräume, die das Hirnwasser (liquor cerebrospinalis) enthalten, vorhanden. Nimmt das Volumen eines dieser Kompartimente zu, muss das Volumen eines oder mehrerer der anderen Kompartimente verdrängt werden, da das Gesamtvolumen des starren Hohlraumes konstant ist. Im gesunden Zustand ist die Volumenelastizität so gross, dass Änderungen des Volumens eines der Kompartimente, z.B. Schwellungen der Hirnsubstanz infolge Verletzungen ohne wesentlichen Druckanstieg ausgeglichen werden können.

Die Volumenelastizität (Compliance) kann mit einem invasiven Untersuchungsverfahren bestimmt werden, bei dem in den Liquorraum über ein Bohrloch im Schädel ein Katheter eingelegt wird, ein bestimmtes Volumen $\Delta V$ einer Flüssigkeit eingespritzt wird und der resultierende Druckanstieg $\Delta p$ gemessen wird. Als Resultat ergibt sich die Compliance C nach der Gleichung $C=\Delta V/\Delta p$ Bei gesunden Menschen ist die Volumenelastizität grösser als 1 ml/mmHg.

Natürlicherweise wird bei jedem Herzschlag ein gewisses Volumen Blut durch die Arterien zugeführt, was einen Druckanstieg verursacht. Dieser Druckanstieg kann mit einer invasiven Druckmesssonde gemessen werden. Allerdings ist das durch den Herzschlag zugeführte Blutvolumen nicht bekannt, so dass die Compliance nicht zahlenmässig ausgerechnet werden kann. Der erfahrene Arzt erhält aber aus der Pulsamplitude des intrakraniellen Druckes (intracranial pressure, ICP) einen Anhalt über die Compliance.

Zur direkten Messung des intrakraniellen Druckes werden miniaturisierte Druckaufnehmer verwendet, die invasiv in den Schädel eingeführt werden. Solche Druckaufnehmer sind als Halbleitersensoren (z. B. gemäss DE000002206624) oder als faseroptische Sensoren (z.B. gemäss US4787396) ausgeführt. Auch die direkte Messung des intrakraniellen Druckes ist eine sehr invasive Prozedur, die für den Patienten eine grosse Belastung und Gefährdung darstellt.

Wegen der Nachteile der direkten Messungen ist auf verschiedene Weise versucht worden, den intrakraniellen Druck und/oder die Compliance nichtinvasiv "von außen" zu bestimmen.

Paulat (DE000019606687A1) beschreibt die Erfassung der elektrischen Impedanz des Schädels mit zwei oder mehreren Elektroden, die an der Kopfhaut angebracht werden. Der Verlauf der Impedanz wird durch das Verhältnis der Kompartimente Blut, Hirnsubstanz und Liquor periodisch bei jedem Herzschlag beeinflusst und es wird eine Kurve mit periodischen Anteilen gewonnen. In dieser Kurve sind charakteristische Punkte (Landmarken) erkennbar, deren Amplitude als charakteristisch für den ICP beschrieben wird. Allerdings ändert sich dieser Zusammenhang von Patient zu Patient durch so vielfältige Einflüsse, dass ein gesichertes diagnostisches Verfahren daraus nicht entwickelt werden konnte.

Yost (US 673773407) beschreibt ein Verfahren, bei dem die Laufzeit eines Ultraschallsignales durch den Schädel hindurch bestimmt wird. Die Geschwindigkeit, mit der der Impuls durch den Inhalt des Schädels läuft, wird durch die unterschiedliche Schallgeschwindigkeit in den Kompartimenten und deren Zusammensetzung beeinflusst. Da die Kalibrierung dieses Verfahrens aber unsicher ist und der Zusammenhang zwischen den Messergebnissen und der zu messenden Grösse von Patient zu Patient durch vielfältige Einflüsse verändert wird, konnte ein gesichertes diagnostisches Verfahren auch daraus nicht entwickelt werden.

Ben-Ari (WO 20141167418) schlägt vor, aus der elektrischen Kapazität und/oder Impedanz des Kopfes eine Kenngrösse für mögliche Ödeme (Wasseransammlungen im Gewebe) im Inneren des Kopfes zu gewinnen. Ebenfalls aufgrund der schwierigen Kalibrierung und der grossen interindividuellen Unterschiede der Situation konnte sich dieses Verfahren nicht durchsetzen.

Weiterhin beschreibt die US 2014/0371545 A1 ein zerebro-hämodynamisches Messgerät mit zumindest einem Prozessor.

Ferner wird in der US 2005/0015009 A1 ein Verfahren zur Bestimmung des ICP eines Subjekts auf der Basis von mindestens zwei variablen Eingängen beschrieben. Weiterhin erwähnen S. Jetzki, M. Kiefer, R. Eymann, M. Walter, und S. Leonhardt [1] im Zusammenhang mit dem dynamischen Verlauf des intrakraniellen Drucks (ICP) die Betrachtung der sogenannten PA-PM slope.

Ähnliche Verfahren sind ebenfalls aus den Dokumenten US 6387051 B1, US 2006/079773 A1 und US 2013/109979 A1 bekannt.

In der Tat werden seit Längerem derartige Kenngrößen betrachtet, die sich aus dem Verhältnis aller spontanen Änderungen der Pulsamplitude zu allen spontanen Änderungen im mittleren intrakraniellen Druck berechnen, siehe z. B. auch die Kenngröße RAP in [2], oder die Kenngröße AMP/P in [3] bzw. die Kenngröße RPPC in [4].

Dem Stand der Technik [1] - [4] ist dabei jedoch gemeinsam, dass alle Änderungen der Pulsamplitude mit allen Änderungen des mittleren Druckes korreliert werden. Die Ursachen der Änderungen der Pulsamplitude können dabei sein:

- Änderung der Pulsamplitude des arteriellen Druckpulses,
- Änderung der Compliance (Nachgiebigkeit des Kopfinhaltes) durch Änderungen des Hirnwasservolumens,
- Änderung der Compliance durch Änderungen des intrakraniellen Blutvolumens vermittels Änderung des Durchmessers der Arterien. (Das Gehirn regelt seine Durchblutung durch Enger- oder Weiterstellen der Arterien in Reaktion auf den Blutdruck. Dieser Mechanismus wird als Autoregulation bezeichnet. Weiterhin regelt das Gehirn seine Durchblutung durch Enger- oder Weiterstellen der Arterien in Reaktion auf den Bedarf. Dieser Mechanismus wird als metabolische Kopplung bezeichnet.),
- Änderungen der Pulsamplitude durch Änderung des Druckes und damit der Compliance durch die Atmung.

Auch die Ursachen der Änderungen des mittleren Druckes sind vielfältiger Natur:

- Änderung des Druckes durch hydrostatische Wirkung bei Lageänderung (Liegen, Sitzen, Stehen),

- Änderung des Druckes durch Änderungen des Hirnwasservolumens,

- Änderung des Druckes durch Änderungen des intrakraniellen Blutvolumens durch Änderung des Durchmessers der Arterien (Autoregulation, metabolische Kopplung).,

- Änderungen des Druckes durch die Atmung.

Da bei den oben zitierten Verfahren all diese Änderungen von Pulsamplitude und Druck miteinander korreliert werden, sind die Korrelationskoeffizienten im klinischen Alltag niedrig und die Kenngrösse hat wenig Aussagekraft. Keines der beschriebenen Systeme zur nichtinvasiven Diagnostik des Hydrozephalus hat sich bisher in der Praxis bewährt.

Aufgabe der vorliegenden Erfindung ist es daher, eine Einrichtung zur Diagnostik des Hydrozephalus und anderer Störungen des Hirndrucks zu schaffen bzw. ein Verfahren, bei der bzw. bei dem aus einem Drucksignal oder einem anderen Signal, das Informationen über den Druck oder die Volumenzusammensetzung im Schädel enthält, eine Information über die Volumenelastizität gewonnen wird.

Die Erfindung macht sich die Tatsache zu Nutze, dass aufgrund des Herzschlages und der Atmung und anderer natürlich vorkommender periodischer Ereignisse periodische Druckschwankungen im ICP vorkommen. Überraschenderweise konnte festgestellt werden, dass sich aus den Verhältnissen dieser Druckschwankungen zueinander dimensionslose Kenngrössen berechnen lassen, die für die Compliance charakteristisch sind. Eine erfindungsgemäss ermittelte Kenngrösse RAQ ist z. B. das Verhältnis der Amplitude Arp der atmungsbedingten Wellen im Drucksignal zur Amplitude AAvp der atmungsbedingten Wellen im Verlauf der herzschlagbedingten Pulsamplitude Avp.

Aus den oben genannten Werten können erfindungsgemäß auch alternative Kenngrößen bzw. Ausgangsgrößen berechnet werden, insbesondere dimensionslosen Kenngrößen, insbesondere in Form von Quotienten bzw. in Form von Kenngrößen, die solche Quotienten aufweisen. Die Berechnung einer erfindungsgemäßen Kenngröße bzw. Ausgangsgröße erfolgt bevorzugt unter Verwendung zumindest einer oder mehrerer der Größen: Avp, Arp, AAvp. Ggf. können weitere Größen zur Bestimmung der Kenngröße (wie z.B. die Herzfrequenz und/oder die Atemfrequenz) verwendet werden. Eine erfindungsgemäße Kenngröße kann natürlich geeignet skaliert bzw. durch Addition oder Subtraktion von z.B. konstanten Werten geeignet verschoben werden. Insbesondere kann die Kenngröße durch eine geeignete Proportionalitätskonstante eine dimensionslosen Kenngröße darstellen.

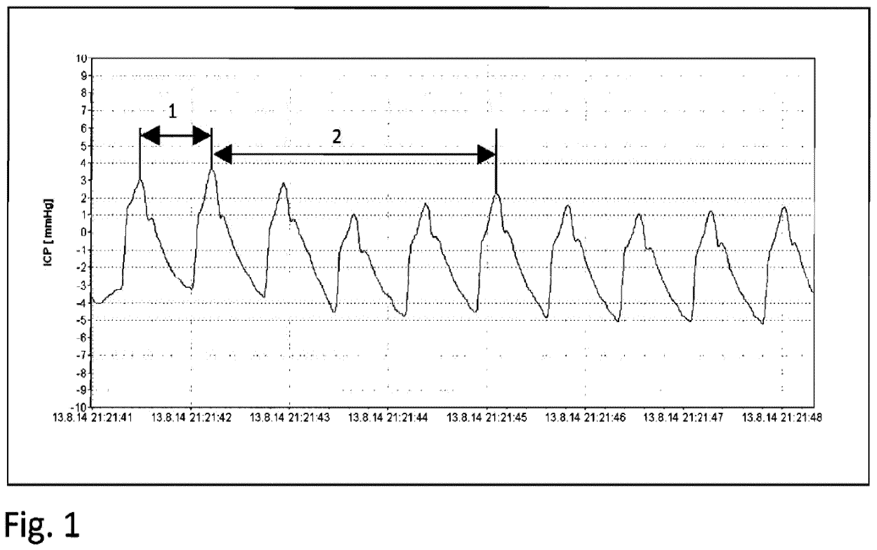

Bei dem ausgewerteten Signal kann es sich, wie im Folgenden beschrieben, um das direkt gemessene Drucksignal handeln. Es kann sich aber auch um ein Impedanz-, Kapazitäts- oder Ultraschallsignal handeln. Ermittelt wird z.B. die Amplitude des Pulses, der dadurch verursacht wird, dass natürlicherweise bei jedem Herzschlag ein gewisses Volumen Blut durch die Arterien zugeführt wird. Weiterhin wird die - niederfrequentere - Druckwelle ermittelt, die dadurch verursacht wird, dass die Blutabfuhr bei jedem Atemzug periodisch gestaut wird, was ebenfalls einer Volumenänderung entspricht, die sich in einer Druckänderung ausdrückt. Da bei der periodischen durch die Atmung verursachten Druckänderung auch die Compliance periodisch absinkt und ansteigt, zeigt sich die Atmung auch in einer Modulation der Amplitude des herzschlagbedingten Pulses. Im Druckverlauf des ICP sind die vom Herzschlag und von der Atmung verursachten Pulsationen erkennbar und messbar. Figur 1 zeigt schematisch den Verlauf des intrakraniellen Druckes mit einem Herzzyklus 1 und einem Atemzyklus 2.

Während die Atmung die herzschlagbedingte - höherfrequente - Pulsamplitude im Bereich der Atemfrequenz moduliert (10 pro Minute bis 20 pro Minute), modulieren andere periodische Einflüsse die Amplitude der herzschlagbedingten Pulswelle (und anderer, jeweils höherfrequenter periodischer Wellen) in anderen Frequenzbereichen: A-Wellen im Bereich von weniger als 1 pro 3 Minuten. B-Wellen (Eigenschwingungen der Regelung der Durchblutung, der sogenannten Autoregulation) im Bereich von 3 pro Minute bis 1 pro 3 Minuten. C-Wellen im Bereich von 3 pro Minute bis 9 pro Minute. Jede der Amplituden der genannten periodischen Wellen wird durch die jeweils niederfrequenteren Wellen moduliert. Im Folgenden werden die Begriffe "modulierte Welle" und "modulierende Welle" verwendet. Wenn also die Amplitude der herzschlagbedingten Pulse durch atmungsbedingte Wellen moduliert wird, handelt es sich bei den herzschlagbedingten Pulsen um die modulierte Welle und bei den atmungsbedingten Wellen um die modulierende Welle. Die folgenden Darstellungen sind zum besseren Verständnis auf die Modulation der Amplitude der herzschlagbedingten Pulswelle durch die Atemwelle bezogen. Sie sind aber bei entsprechender Änderung der frequenzbestimmenden Komponenten auch auf beliebige andere Kombinationen von modulierender und modulierter Welle anwendbar.

Bei dem Verhältnis RAQ aus der Amplitude $A_{rp}$ der atmungsbedingten Wellen im ausgewerteten Signal zur Amplitude $AA_{vp}$ der atmungsbedingten Wellen im Verlauf der herzschlagbedingten Pulsamplitude $A_{vp}$ des ausgewerteten Signals handelt es sich um eine Verhältniszahl, die nach der Gleichung $RAQ = A_{rp} / AA_{vp}$ ausgerechnet wird. Sie ist unabhängig von der Kalibrierung. Deshalb ist die erfindungsgemässe Einrichtung besonders geeignet zur Auswertung von nichtinvasiv gewonnen Signalen, die Informationen über den Druck oder die Volumenzusammensetzung oder die Volumenelastizität im Schädel enthalten. Auf diese Weise ist es erstmals möglich, aus unkalibrierten Signalen gesicherte Informationen über die intrakranielle Compliance auf nichtinvasive Weise zu erhalten. Weiterhin ist es erstmals möglich aus dem invasiv direkt gemessenen Drucksignal gesicherte Informationen über die intrakranielle Compliance zu gewinnen, ohne dass eine Substanz eingespritzt werden muss.

Erfindungsgemäß ist eine Einrichtung zur Bestimmung einer Kenngröße des Kopfinhaltes von Menschen und anderen Säugetieren vorgesehen, die zur Gewinnung und Auswertung eines für den Kopfinhalt charakteristischen Signales als Eingangsgröße vorgesehen ist, wobei die Einrichtung dazu konfiguriert ist,

    a. die Pulsamplitude $A_{vp}$ jeder einzelnen Periode (oder Halbperiode) einer modulierten Welle des charakteristischen Signals auszurechnen und ein Signal $A_{vp}$ oder eine Zeitreihe $A_{vp}$ zu erzeugen,
    b. den mittleren Wert MICP für jede einzelne Periode (oder Halbperiode) der modulierten Welle des charakteristischen Signals zu berechnen und ein Signal MICP oder eine Zeitreihe MICP zu erzeugen,
    c. die Amplitude $A_{rp}$ einer modulierenden Welle in der Zeitreihe MICP oder in dem Signal MICP zu berechnen,
    d. die Amplitude $AA_{vp}$ der modulierenden Welle in der Zeitreihe $A_{vp}$ oder in dem Signal $A_{vp}$ zu berechnen,
    e. die Kenngröße als Ausgangsgröße unter Verwendung zumindest einer oder mehrerer der Größen: $A_{vp}$, $A_{rp}$, $AA_{vp}$ zu berechnen, wobei die Kenngröße ein Quotient ist, der die Größen $A_{rp}$ und $A_{vp}$ aufweist.

Bei dem charakteristischen Signal handelt es sich um eines der folgenden Signale: die elektrische Impedanz des Schädels, der direkt gemessene Druck im Inneren des Kopfes, die elektrische Kapazität des Schädels, die Laufzeit eines durch den Schädel geleiteten Ultraschallsignales.

Gemäß einer Ausführungsform der Erfindung ist vorgesehen, dass die Kenngröße eine dimensionslose Kenngröße ist.

Die erfindungsgemäße Lösung beseitigt damit insbesondere die Schwäche der oben zitierten Ansätze [1] bis [4], indem sowohl bei der Änderung des Pulsamplitude, als auch der Änderung des mittleren Druckes (MICP) nur ein Einfluss (z. B. die Atmung) berücksichtigt wird. Insbesondere werden ja nur die Änderungen der Amplitude einer modulierten Welle in einem schmalen Frequenzband und die Änderungen der Amplitude einer modulierenden Welle im gleichen schmalen Frequenzband betrachtet. Es werden z. B. hochselektiv sowohl im Zähler, als auch im Nenner des Quotienten RAQ nur periodische Änderungen im gleichen schmalen Frequenzbereich betrachtet.

Bei erfindungsgemäßer Anwendung auf die Atemwelle als modulierende Welle und die Pulswelle als modulierte Welle werden z. B. nur die Änderungen der Pulsamplitude im Atemfrequenzbereich als modulierte Welle und die Änderungen des mittleren Druckes im Atemfrequenzbereich als modulierende Welle berücksichtigt. Durch die schmalbandige Begrenzung auf z. B. den Atemfrequenzbereich wird also bei der Kenngröße, insbesondere sowohl im Zähler, als auch im Nenner, nur ein Einfluss berücksichtigt, während alle durch Lageänderung, Autoregulation usw. verursachten Schwankungen herausgefiltert sind.

Der oben genannte Schritt a) ist demnach insbesondere so zu verstehen, dass die Pulsamplitude $A_{vp}$ jeder einzelnen Periode (oder Halbperiode) genau einer bzw. lediglich einer modulierten Welle des charakteristischen Signals ausgerechnet wird und ein Signal $A_{vp}$ oder eine Zeitreihe $A_{vp}$ erzeugt wird,

Weiterhin ist entsprechend des oben genannten Schritts c) demnach insbesondere so zu verstehen, dass die Amplitude $A_{rp}$ genau einer bzw. lediglich einer modulierenden Welle in der Zeitreihe MICP oder in dem Signal MICP berechnet wird. Weiterhin ist gemäß einer Ausführungsform der Erfindung vorgesehen, dass die Kenngröße ein Quotient ist oder einen Quotienten aufweist.

Weiterhin ist gemäß einer Ausführungsform der Erfindung vorgesehen, dass die Kenngröße ein Quotient ist, der die Größen $A_{rp}$ und $AA_{vp}$ aufweist.

Weiterhin ist gemäß einer Ausführungsform der Erfindung vorgesehen, dass die Kenngröße ein Quotient ist, der die Größe HF und/oder AF aufweist, wobei HF die Herzfrequenz und AF die Atemfrequenz ist.

Weiterhin ist gemäß einer Ausführungsform der Erfindung vorgesehen, dass der Quotient RAQ proportional zu oder gleich einem der folgenden Ausdrücke ist: $A_{rp}/AA_{vp}$, $A_{rp}/(AAvp/AF)$, $A_{rp}/(AA_{vp}*HF)$, $A_{rp}/(AA_{vp}*(HF/AF))$.

Weiterhin ist gemäß einer Ausführungsform der Erfindung vorgesehen, dass das charakteristische Signal der direkt gemessene Druck im Inneren des Kopfes ist (ICP).

Weiterhin ist gemäß einer Ausführungsform der Erfindung vorgesehen, dass das charakteristische Signal die elektrische Impedanz des Schädels ist, die mittels außen am Schädel anbringbaren Elektroden aufnehmbar ist.

Weiterhin ist gemäß einer Ausführungsform der Erfindung vorgesehen, dass das charakteristische Signal die elektrische Kapazität des Schädels ist, die mittels außen am Schädel anbringbaren Elektroden aufnehmbar ist.

Die erfindungsgemäße Einrichtung kann die besagten Elektroden aufweisen, wobei diese gegebenenfalls mit der oder den Einheiten der erfindungsgemäßen Einrichtung verbunden sind, sodass die besagte Impedanz bzw. Kapazität mittels der Einrichtung verarbeitet werden kann.

Weiterhin ist gemäß einer Ausführungsform der Erfindung vorgesehen, dass das charakteristische Signal die Laufzeit eines Ultraschallsignales ist, das durch den Schädel geleitet wird.

Weiterhin ist gemäß einer Ausführungsform der Erfindung vorgesehen, dass das charakteristische Signal die Laufzeit eines Ultraschallsignales ist, das aus dem Inneren des Schädels reflektiert wird.

Weiterhin ist gemäß einer Ausführungsform der Erfindung vorgesehen, dass die Einrichtung eine oder mehrere Einheiten aufweist, die dazu konfiguriert ist bzw. sind

    a. die Pulsamplitude $A_{vp}$ jeder einzelnen Periode (oder Halbperiode) der modulierten Welle des charakteristischen Signals auszurechnen und ein Signal $A_{vp}$ oder eine Zeitreihe $A_{vp}$ zu erzeugen (3),

    b. den mittleren Wert MICP für jede einzelne Periode (oder Halbperiode) der modulierten Welle des charakteristischen Signals zu berechnen und ein Signal MICP oder eine Zeitreihe MICP zu erzeugen (4),

    c. die Amplitude $A_{rp}$ einer modulierenden Welle in der Zeitreihe MICP oder in dem Signal MICP zu berechnen (5),

    d. die Amplitude $AA_{vp}$ der modulierenden Welle in der Zeitreihe $A_{vp}$ oder in dem Signal $A_{vp}$ zu berechnen (6),

    e. die Kenngröße als Ausgangsgröße unter Verwendung zumindest einer oder mehrerer der Größen: $A_{vp}$, $A_{rp}$, $AA_{vp}$ zu berechnen.

Insbesondere kann für jeden der vorbezeichneten Berechnungen bzw. Schritte a bis e eine separate Einheit vorgesehen sein. Es ist aber auch denkbar, dass die einzelnen Berechnungen/Schritte durch eine Einheit vorgenommen werden oder durch mehrere Einheiten, auf die die Berechnungen geeignet verteilt werden. Diejenige Einheit, die die Berechnung der Kenngröße vornimmt kann natürlich auch sämtliche andere hierin offenbarten Varianten der Kenngrößenberechnung vornehmen.

Weiterhin ist gemäß einer Ausführungsform der Erfindung vorgesehen, dass die jeweilige Einheit eine analoge Rechenschaltung, eine digitale Rechenschaltung oder eine programmgesteuerte Mikroprozessorschaltung ist.

Gemäß einer Ausführungsform kann die modulierte Welle eine der periodischen Signale aus der Gruppe der B-Wellen (1/3 Periode pro min bis 3 Perioden pro min), C-Wellen (3 Perioden pro min bis 9 Perioden pro min), Atemwellen (10 Perioden pro min bis 20 Perioden pro min), herzschlagbedingte Wellen (20 Perioden pro min bis 240 Perioden pro min) sein.

Weiterhin kann gemäß einer Ausführungsform die modulierende Welle eine der periodischen Signale aus der Gruppe der A-Wellen (< 1 pro 4 min), B-Wellen (1/3 pro min bis 3 pro min), C-Wellen (3 pro min bis 9 pro min), Atemwellen (10 pro min bis 20 pro min) sein, welche niederfrequenter als die modulierte Welle ist.

Gemäß einer weiteren Ausführungsform wird eine Einrichtung offenbart, die dazu konfiguriert die besagte Kenngröße als erste Ausgangsgröße zu berechnen, und die zusätzlich dazu konfiguriert ist eine weitere Kenngröße als zweite Ausgangsgröße zu berechnen, wobei zur Berechnung der weiteren Ausgangsgröße insbesondere eine Kombination von modulierter und modulierender Welle verwendet wird wird, die sich von der zur Berechnung der Kenngröße

## EP 3 621 513 B1

bzw. ersten Ausgangsgröße verwendeten Kombination von modulierter und modulierender Welle unterscheidet, wobei die Einrichtung hier dazu konfiguriert ist, als Kenngröße des Kopfinhaltes den Quotienten aus der ersten Ausgangsgröße und der zweiten Ausgangsgröße zu bilden.

In dieser Ausführungsform kann die Einrichtung z.B. zwei erfindungsgemäße Einrichtungen aufweisen, wobei die eine Einrichtung die erste Ausgangsgröße und die andere Einrichtung die zweite Ausgangsgröße berechnet. Die Einrichtung berechnet daraus dann den besagten Quotienten aus der ersten und der zweiten Ausgangsgröße.

Weiterhin ist gemäß einer Ausführungsform vorgesehen, dass die Einrichtung dazu konfiguriert ist, die Zeitreihe $A_{vp}$ zu bilden, indem für den Zeitabschnitt zwischen einem lokalen Minimum und dem darauf folgenden lokalen Maximum der modulierten Welle des charakteristischen Signals die Differenz zwischen dem lokalen Maximum und dem lokalen Minimum als Wert für die Zeitreihe $A_{vp}$ verwendet wird, und indem für den darauf folgenden Zeitabschnitt zwischen dem lokalen Maximum und dem darauf folgenden lokalen Minimum der modulierten Welle des charakteristischen Signals entsprechend die Differenz zwischen dem lokalen Maximum und dem darauf folgenden lokalen Minimum als Wert der Zeitreihe $A_{vp}$ verwendet wird.

Weiterhin ist gemäß einer Ausführungsform vorgesehen, dass die Einrichtung dazu konfiguriert ist, die Zeitreihe MICP zu bilden, indem für den Zeitabschnitt zwischen einem lokalen Minimum und dem darauf folgenden lokalen Maximum der modulierten Welle des charakteristischen Signals der mittlere Wert zwischen dem lokalen Maximum und dem lokalen Minimum als Wert für die Zeitreihe MICP verwendet wird, und indem für den darauf folgenden Zeitabschnitt zwischen dem lokalen Maximum und dem darauf folgenden lokalen Minimum der modulierten Welle des charakteristischen Signals entsprechend der mittlere Wert zwischen dem lokalen Maximum und dem darauf folgenden lokalen Minimum als Wert der Zeitreihe MICP verwendet wird.

Ein weiterer Aspekt der vorliegenden Erfindung betrifft ein Verfahren zur Bestimmung einer Kenngröße des Kopfinhaltes von Menschen und anderen Säugetieren, unter Verwendung einer erfindungsgemäßen Einrichtung, aufweisend die Schritte:

a. Ausrechnung der Pulsamplitude $A_{vp}$ jeder einzelnen Periode (oder Halbperiode) einer modulierten Welle des charakteristischen Signals und Erzeugung eines Signales $A_{vp}$ oder einer Zeitreihe $A_{vp}$ (3),
b. Berechnung des mittleren Wertes MICP für jede einzelne Periode (oder Halbperiode) der modulierten Welle des charakteristischen Signals und Erzeugung eines Signales MICP oder einer Zeitreihe MICP (4),
c. Berechnung der Amplitude $A_{rp}$ einer modulierenden Welle in der Zeitreihe MICP oder in dem Signal MICP (5),
d. Berechnung der Amplitude $AA_{vp}$ der modulierenden Welle in der Zeitreihe $A_{vp}$ (6),
e. Berechnung der Kenngröße unter Verwendung zumindest einer oder mehrerer der Größen: $A_{vp}$, $A_{rp}$, $AA_{vp}$.

Gemäß einer Ausführungsform des erfindungsgemäßen Verfahrens ist vorgesehen, dass die Kenngröße eine dimensionslose Kenngröße ist.

Weiterhin ist gemäß einer Ausführungsform des erfindungsgemäßen Verfahrens vorgesehen, dass die Kenngröße ein Quotient ist oder einen Quotienten aufweist.

Weiterhin ist gemäß einer Ausführungsform des erfindungsgemäßen Verfahrens vorgesehen, dass die Kenngröße ein Quotient ist, der die Größen $A_{rp}$ und $AA_{vp}$ aufweist.

Weiterhin ist gemäß einer Ausführungsform des erfindungsgemäßen Verfahrens vorgesehen, dass die Kenngröße ein Quotient ist, der die Größe HF und/oder AF aufweist, wobei HF die Herzfrequenz und AF die Atemfrequenz ist.

Weiterhin ist gemäß einer Ausführungsform des erfindungsgemäßen Verfahrens vorgesehen, dass der Quotient RAQ proportional zu oder gleich einem der folgenden Ausdrücke ist: $A_{rp}/AA_{vp}$, $A_{rp}/(AAvp/AF)$, $A_{rp}/(AA_{vp}*HF)$, $A_{rp}/(AA_{vp}*(HF/AF))$.

Weiterhin ist gemäß einer Ausführungsform des erfindungsgemäßen Verfahrens vorgesehen, dass das charakteristische Signal der direkt gemessene Druck im Inneren des Kopfes ist.

Weiterhin ist gemäß einer Ausführungsform des erfindungsgemäßen Verfahrens vorgesehen, dass das charakteristische Signal die elektrische Impedanz des Schädels ist, die mittels außen am Schädel angebrachten Elektroden aufgenommen wird.

Weiterhin ist gemäß einer Ausführungsform des erfindungsgemäßen Verfahrens vorgesehen, dass das charakteristische Signal die elektrische Kapazität des Schädels ist, die mittels außen am Schädel angebrachter Elektroden aufgenommen wird.

Weiterhin ist gemäß einer Ausführungsform des erfindungsgemäßen Verfahrens vorgesehen, dass das charakteristische Signal die Laufzeit eines Ultraschallsignales ist, das durch den Schädel geleitet wird.

Weiterhin ist gemäß einer Ausführungsform des erfindungsgemäßen Verfahrens vorgesehen, dass das charakteristische Signal die Laufzeit eines Ultraschallsignales ist, das aus dem Inneren des Schädels reflektiert wird.

Weiterhin ist gemäß einer Ausführungsform des erfindungsgemäßen Verfahrens vorgesehen, dass die Zeitreihe $A_{vp}$ gebildet wird, indem für den Zeitabschnitt zwischen einem lokalen Minimum und dem darauf folgenden lokalen Maximum der modulierten Welle des charakteristischen Signals die Differenz zwischen dem lokalen Maximum und dem lokalen Minimum als Wert für die Zeitreihe $A_{vp}$ verwendet wird, und indem für den darauf folgenden Zeitabschnitt zwischen dem lokalen Maximum und dem darauf folgenden lokalen Minimum der modulierten Welle des charakteristischen Signals entsprechend die Differenz zwischen dem lokalen Maximum und dem darauf folgenden lokalen Minimum als Wert der Zeitreihe $A_{vp}$ verwendet wird.

Weiterhin können bei dem erfindungsgemäßen Verfahren auch zwei erfindungsgemäße Einrichtung verwendet werden (siehe oben) bzw. zwei Ausgangsgrößen berechnet werden, wobei die erste Einrichtung bzw. Ausgangsgröße durch eine bestimmte Kombination von modulierter und modulierender Welle gekennzeichnet ist, und wobei die zweite Einrichtung bzw. Ausgangsgröße durch eine andere Kombination von modulierter und modulierender Welle gekennzeichnet ist, und wobei die Kenngröße des Kopfinhaltes durch den Quotienten aus der ersten Ausgangsgröße und der zweiten Ausgangsgröße gebildet wird. Für die besagten Kombinationen von modulierter und modulierender Welle können insbesondere alle hierin beschriebenen Kombinationen bzw. Beispiele verwendet werden.

Weitere Merkmale, Ausführungsformen und Vorteile der vorliegenden Erfindung sollen nachfolgend anhand von Figuren erläutert werden. Es zeigen

Fig. 1      eine Darstellung des Verlaufs des intrakraniellen Druckes mit einem Herzzyklus 1 und einem Atemzyklus 2;

Fig. 2      ein Blockdiagramm einer erfindungsgemäßen Einrichtung bzw. eines erfindungsgemäßen Verfahrens; und

Fig. 3A-3J      zeigen exemplarisch die Auswertung des Drucksignals ICP im Rahmen der vorliegenden Erfindung.

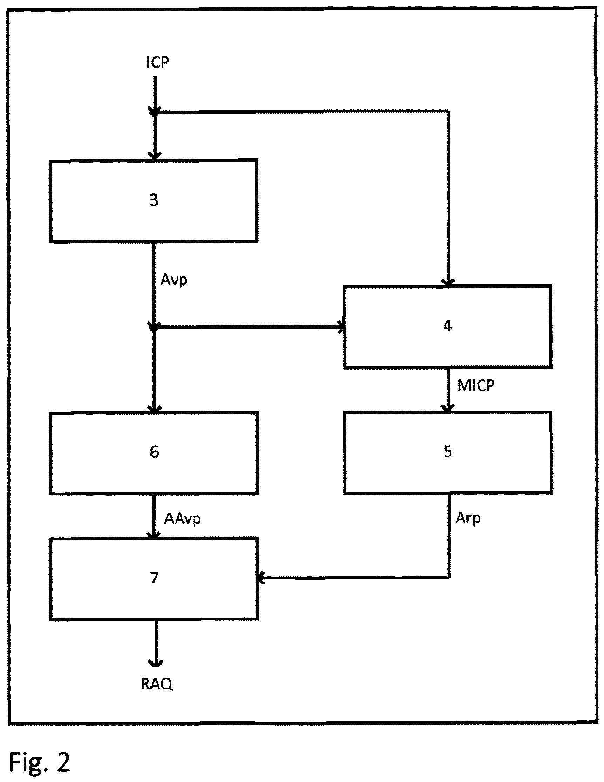

Eine erfindungsgemäße Einrichtung bzw. ein erfindungsgemäßes Verfahren sind in der Fig. 2 schematisch dargestellt.

Gemäß einer Ausführungsform der Erfindung besteht die Einrichtung, wie in Fig. 2 beispielhaft für die Auswertung des ICP als Blockschaltbild gezeigt, aus einer Auswerteeinheit für ein invasiv oder nichtinvasiv gewonnenes charakteristisches Signal, die Einheiten für folgende Auswertungen enthält:

a. Ausrechnung der Pulsamplitude Avp jedes einzelnen Herzschlages in dem charakteristischen Signal und Erzeugung eines Signales Avp oder einer Zeitreihe Avp (3).

b. Berechnung des mittleren Wertes MICP des charakteristischen Signals für jeden einzelnen Herzschlag und Erzeugung eines Signales MICP oder einer Zeitreihe MICP (4).

c. Berechnung der Amplitude Arp der Atemwelle in dem erzeugten Signal MICP oder in der Zeitreihe MICP (5)

d. Berechnung der Amplitude AAvp der Atemwelle in dem erzeugten Signal Avp oder in der Zeitreihe Avp (6).

e. Berechnung des Quotienten der Kenngröße als eine Größe RAQ die proportional ist zu $RAQ = A_{rp} / AA_{vp}$ (7), oder Berechnung der Kenngröße in Form des Quotienten RAQ, wobei $RAQ = A_{rp} / AA_{vp}$ (7).

In dieser Ausführung der Erfindung ist die herzschlagbedingte Welle die modulierte Welle und die atmungsbedingte Welle die modulierende Welle. Analog dazu ist jede Kombination aus höherfrequenterer modulierter Welle und niederfrequenterer modulierender Welle möglich.

Überraschenderweise wurde festgestellt, dass die Ausrechnung der Pulsamplitude Avp als Mittelwert der Differenz zweier lokaler Maxima der modulierten Welle des charakteristischen Signals zu dem zwischen diesen beiden Maxima liegenden lokalen Minimum der modulierten Welle des charakteristischen Signals zu systematisch durch die modulierende Welle verfälschten Amplitudenwerten führt.

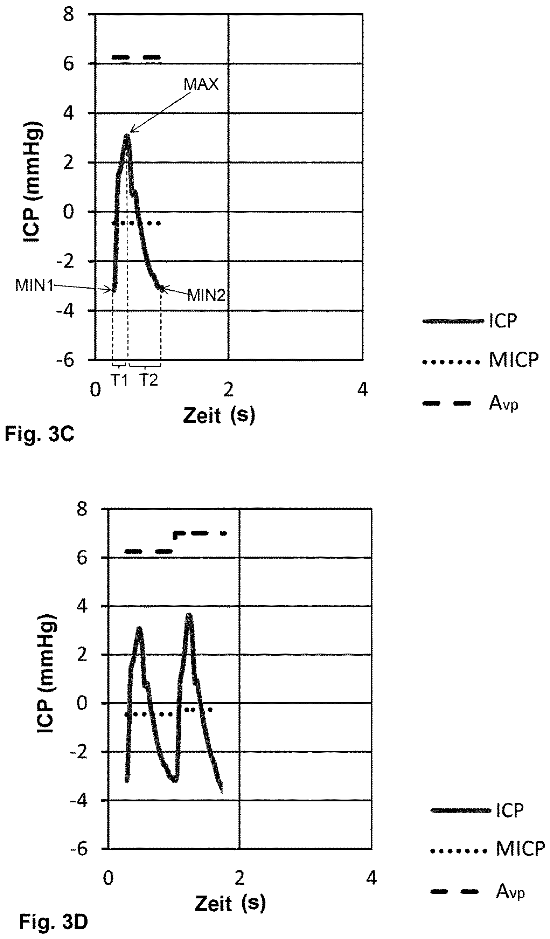

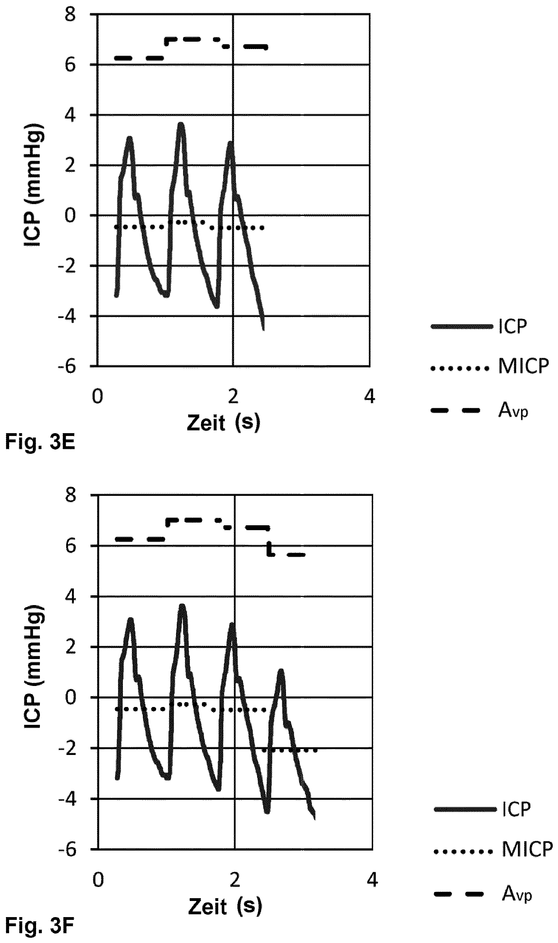

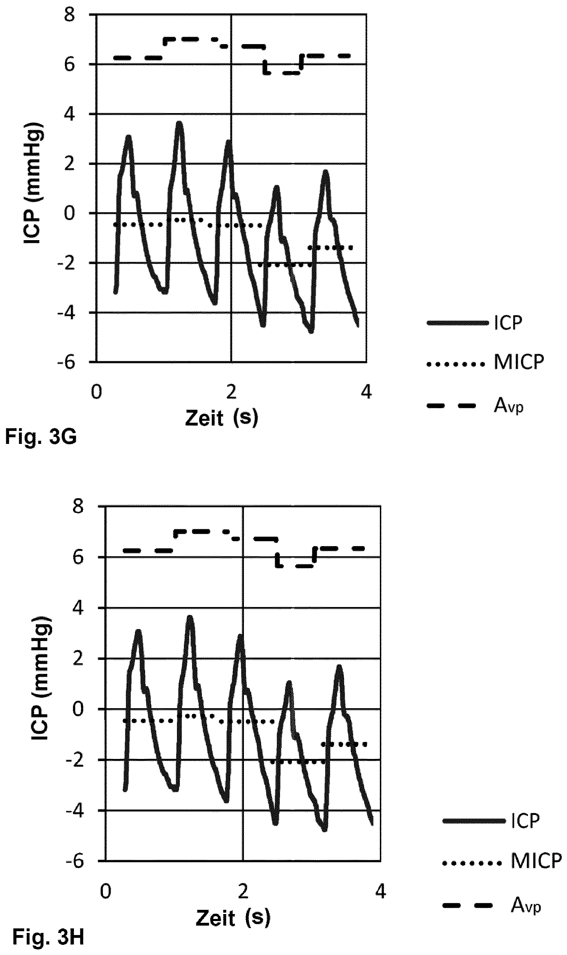

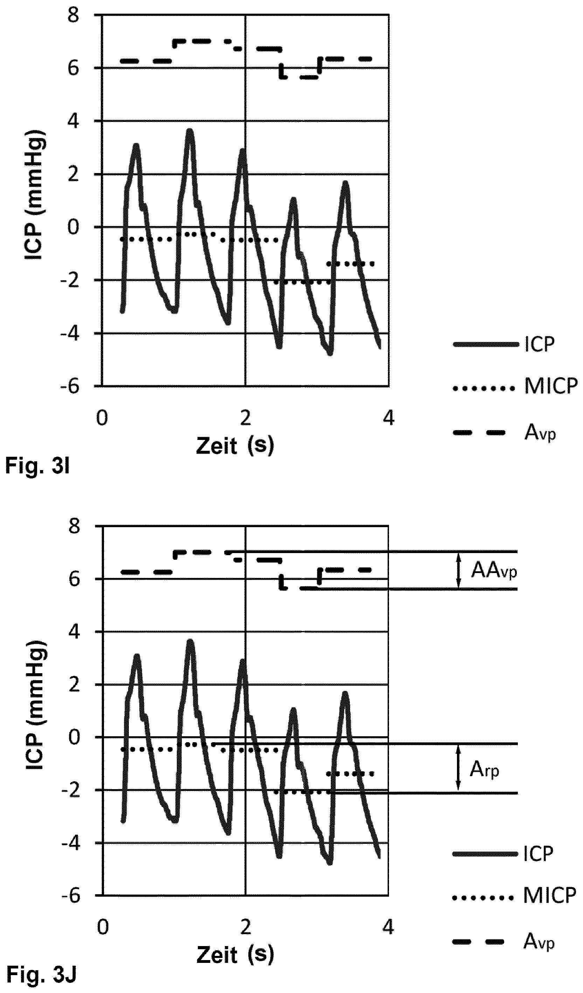

Deshalb wird zur Berechnung der Pulsamplitude $A_{vp}$ im Rahmen der vorliegenden Erfindung vorzugsweise für den Zeitabschnitt zwischen einem lokalen Minimum und dem darauf folgenden lokalen Maximum der modulierten Welle des charakteristischen Signals die Differenz zwischen dem lokalen Maximum und dem lokalen Minimum gewählt und für den darauf folgenden Zeitabschnitt zwischen dem lokalen Maximum und dem darauf folgenden lokalen Minimum entsprechend die Differenz zwischen dem lokalen Maximum und dem darauf folgenden lokalen Minimum. Dies wird weiter unten anhand der Figur 3C exemplarisch erläutert.

Da das Herzschlagvolumen bei zunehmender Herzfrequenz und gleicher Durchblutung abnimmt, ist gemäß einer Ausführungsform der Erfindung vorgesehen, dass in der Kenngröße bzw. in dem Quotienten RAQ die Amplitude AAvp mit der Herzfrequenz oder mit einer aus ihr abgeleiteten Größe multipliziert wird, so dass die Kenngröße RAQ proportional ist zu

$$Arp \ / \ (AA_{vp}*HF),$$

oder so dass die Kenngröße RAQ gegeben ist durch

$$RAQ = A_{rp} \ / \ (AA_{vp}*HF),$$

wobei mit HF die Herzfrequenz gemeint ist. Dadurch wird RAQ unabhängig vom Herzschlagvolumen.

Weiterhin ist gemäß einer Ausführungsform der Erfindung vorgesehen, dass auch die Atemfrequenz AF verrechnet werden kann (weil bei hohen Atemfrequenzen die Änderungsrate pro Zeit höher ist). Dementsprechend besteht die Möglichkeit, die Kenngröße bzw. den besagten Quotienten RAQ durch die Atemfrequenz zu korrigieren, indem bei der Berechnung der Kenngröße bzw. des Quotienten RAQ die Amplitude $AA_{vp}$ durch die Atemfrequenz AF geteilt wird oder durch eine aus der Atemfrequenz AF abgeleitete Größe geteilt wird.

Entsprechend ist dann die Kenngröße RAQ proportional zu

$$A_{rp} \ / \ (AA_{vp}/AF)$$

oder ergibt sich als Quotient

$$RAQ = A_{rp} \ / \ (AA_{vp}/AF)$$

Hierdurch wird die Kenngröße bzw. der Quotient RAQ unabhängig von der Atemfrequenz.

Die beiden vorstehend genannten Korrekturmöglichkeiten können natürlich gemäß einer weiteren Ausführungsform auch kombiniert werden. Die besagte Kenngröße (hier der Quotient RAQ) berechnet sich dann zu

$$RAQ = A_{rp}/(AA_{vp}*(HF/AF)),$$

oder ist proportional zu $A_{rp}/(AA_{vp}*(HF/AF))$.

Die oben genannten Formeln zur Berechnung von RAQ können ggf. auf der rechten Seite einen Proportionalitätsfaktor enthalten, so dass ggf. die errechnete Kenngröße bzw. der Quotient RAQ insbesondere dimensionslos ist. Dies ist insbesondere der Fall, wenn lediglich mit der Größe HF oder AF korrigiert wird, die jeweils die Dimension einer Frequenz aufweisen.

Sofern die Atemfrequenz AF und/oder die Herzfrequenz zur Bestimmung der Kenngröße verwendet wird, kann die erfindungsgemäße Einrichtung eine Einheit zur Messung der Herzfrequenz und/oder eine Einheit zur Messung der Atemfrequenz aufweisen.

Bei der Berechnung des Quotienten RAQ gemäß dem Ausführungsbeispiel der Figur 2 kann das Multiplizieren des Wertes AAvp mit der Herzfrequenz HF und oder das Teilen durch die Atemfrequenz nach Schritt 6, also nach der Bestimmung von $AA_{vp}$ vorgenommen werden.

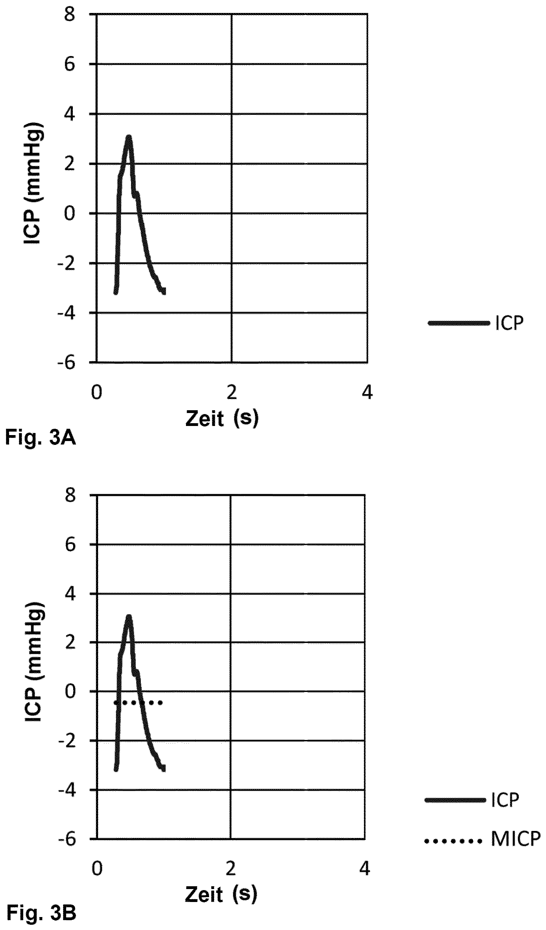

Fig. 3A bis 3J zeigen, ebenfalls beispielhaft für die Auswertung des ICP, wie die einzelnen Werte aus dem Drucksignal ermittelt werden.

Fig. 3A zeigt zunächst eine Periode des Drucksignals ICP. In der Fig. 3B ist ersichtlich, wie hieraus der Mittelwert MICP der gezeigten Periode von ICP berechnet wird. Weiterhin zeigt die Fig. 3C, wie die Pulsamplitude $A_{vp}$ berechnet werden kann, nämlich z.B. indem für den Zeitabschnitt T1 zwischen dem ersten lokalen Minimum MIN1 von ICP und dem ersten lokalen Maximum MAX von ICP eine Differenz zwischen dem lokalen Maximum MAX von ICP und dem ersten lokalen Minimum MIN1 von ICP gebildet wird ($A_{vp}$(T1)=MAX-MIN1). Für den zweiten Zeitabschnitt T2 zwischen dem lokalen Maximum MAX von ICP und dem zweiten lokalen Minimum MIN2 von ICP kann für $A_{vp}$ entsprechend die Differenz zwischen dem lokalen Maximum MAX von ICP und dem zweiten Minimum MIN2 von ICP angesetzt werden (also $A_{vp}$(T2)=MAX-MIN2). Sinngemäss wird dann MICP ebenfalls separat für die Halbperioden T1 und T2 bestimmt als mittlerer Druck zwischen MIN1 und MAX sowie zwischen MAX und MIN2. Dies kann bei den weiteren Perioden von ICP, die in den Figuren 3D bis 3I gezeigt sind, fortgeführt werden. Sinngemäss können $A_{vp}$ und MICP jeweils für "Halbperioden" zwischen lokalem Minimum und lokalem Maximum als erster Halbperiode und zwischen lokalem Maximum und nächstem lokalem Minimum als zweiter Halbperiode bestimmt werden, oder sie können jeweils für ganze Perioden bestimmt werden.

Fig. 3J zeigt dann beispielhaft die Berechnung der Amplitude $AA_{vp}$ aus der Zeitreihe $A_{vp}$ sowie die Berechnung der Amplitude $A_{rp}$ aus der Zeitreihe MICP. Hierbei ergibt sich $AA_{vp}$ als Differenz zwischen dem Maximum und dem Minimum der Zeitreihe $A_{vp}$. Weiterhin ergibt sich $A_{rp}$ als Differenz zwischen dem Maximum und dem Minimum der Zeitreihe MICP.

Der sich ergebende Wert RAQ ist dimensionslos und unabhängig von der Kalibrierung und dem Übertragungsfaktor des gewonnen Signales. Deshalb ist er besonders geeignet zum Einsatz mit nichtinvasiv gewonnen Signalen, deren Kalibrierung und Übertragungsfaktoren nicht bekannt sind.

In einer bevorzugten Ausführung der Erfindung wird die oben beschriebene Auswertung durch eine oder mehrere analoge Rechenschaltungen vorgenommen.

In einer weiteren bevorzugten Ausführung der Erfindung wird die Auswertung durch eine oder mehrere digital arbeitende Rechenschaltungen vorgenommen.

In einer besonders bevorzugten Ausführung der Erfindung wird die Auswertung durch eine oder mehrere Mikroprozessorschaltungen vorgenommen, die durch ein oder mehrere Programme gesteuert werden. Weiterhin wird die Berechnung der unter a) beschriebenen Pulsamplitude und der unter a) und b) beschriebenen Signale oder Zeitreihen durch einen Algorithmus im Zeitbereich durchgeführt. Bevorzugt wird die Berechnung der unter c) und d) beschriebenen Amplituden durch Bestimmung der Maxima und Minima in den Zeitreihen $A_{vp}$ und $AA_{rp}$ und Bildung der Differenzen zwischen aufeinanderfolgenden Maxima und Minima durchgeführt.

Besonders bevorzugt wird die Berechnung der unter c) und d) beschriebenen Amplituden durch Berechnung der Amplitudenspektren der Zeitreihen $A_{vp}$ und $AA_{rp}$ und Bestimmung der Maxima im Bereich der Atemfrequenz durchgeführt.

In einer weiteren besonderen Ausführung der Erfindung sind zwei erfindungsgemäße Einrichtungen für verschiedene Kombinationen von modulierter und modulierender Welle vorhanden und die Kenngrösse wird durch Bildung des Quotienten aus der Ausgangsgrösse oder dem Ausgangssignal der ersten Einrichtung und der Ausgangsgrösse oder dem Ausgangssignal der zweiten Einrichtung gebildet. Bevorzugt wird in der ersten Einrichtung das Signal RAQ1 mit der Atemwelle als modulierender Welle und der herzschlagbedingten Pulswelle als modulierter Welle gebildet, in der zweiten Einrichtung wird das Signal RAQ2 mit der B-Welle als modulierender Welle und der herzschlagbedingten Pulswelle als modulierter Welle gebildet, wobei die Kenngrösse als Quotient RAQ1/RAQ2 oder dessen Kehrwert gebildet wird.

Literatur

[1] S. Jetzki, M. Kiefer, R. Eymann, M. Walter, und S. Leonhardt, "Analysis of pulse waves in intracranial pressure" in Conference proceedings: Annual International Conference of the IEEE Engineering in Medicine and Biology Society. IEEE Engineering in Medicine and Biology Society. Conference, 2006, vol. 2007, pp. 2863-2866.

[2] D. J. Price, M. Czosnyka, and M. Williamson, "Attempts to continuously monitor autoregulation and compensatory reserve in severe head injuries," in Intracranial Pressure VIII, Springer, 1993, pp. 61-66,

[3] M. Czosnyka, Z. Czosnyka, N. Keong, A. Lavinio, P. Smielewski, S. Momjian, E. A. Schmidt, G. Petrella, B. Owler, and J. D. Pickard, "Pulse pressure waveform in hydrocephalus: what it is and what it isn't," Neurosurg. Focus, vol. 22, no. 4, pp. 1-7, 2007.

[4] N. Lenfeldt, N. Andersson, A. Ågren-Wilsson, A. T. Bergenheim, L.-O. D. Koskinen, A. Eklund, and J. Malm, "Cerebrospinal fluid pulse pressure method: a possible substitute for the examination of B waves," J. Neurosurg., vol. 101, no. 6, pp. 944-950, 2004.

**Patentansprüche**

1. Einrichtung zur Bestimmung einer Kenngröße des Kopfinhaltes von Menschen und anderen Säugetieren, mit einer Gewinnung und Auswertung eines für den Kopfinhalt charakteristischen Signales als Eingangsgröße, wobei das charakteristische Signal eines der folgenden Signale ist: die elektrische Impedanz des Schädels, der direkt gemessene Druck im Inneren des Kopfes, die elektrische Kapazität des Schädels, die Laufzeit eines durch den Schädel geleiteten Ultraschallsignales, und wobei die Einrichtung dazu konfiguriert ist,

a. die Pulsamplitude $A_{vp}$ jeder einzelnen Periode oder Halbperiode einer modulierten Welle des charakteristischen Signals auszurechnen und ein Signal $A_{vp}$ oder eine Zeitreihe $A_{vp}$ zu erzeugen (3),
b. den mittleren Wert MICP für jede einzelne Periode oder Halbperiode der modulierten Welle des charakteristischen Signals zu berechnen und ein Signal MICP oder eine Zeitreihe MICP zu erzeugen (4),
c. die Amplitude $A_{rp}$ einer modulierenden Welle in der Zeitreihe MICP oder in dem Signal MICP zu berechnen (5),

d. die Amplitude $AA_{vp}$ der modulierenden Welle in der Zeitreihe $A_{vp}$ oder in dem Signal $A_{vp}$ zu berechnen (6),
e. die Kenngröße als Ausgangsgröße unter Verwendung zumindest einer oder mehrerer der Größen: $A_{vp}$, $A_{rp}$, $AA_{vp}$ zu berechnen, wobei die Kenngröße ein Quotient ist, der die Größen $A_{rp}$ und $AA_{vp}$ aufweist.

2. Einrichtung nach Anspruch 1, **dadurch gekennzeichnet, dass** die Kenngröße eine dimensionslose Kenngröße ist.

3. Einrichtung nach einem der vorhergehenden Ansprüche, **dadurch gekennzeichnet, dass** die Kenngröße ein Quotient ist, der die Größe HF und/oder AF aufweist, wobei HF die Herzfrequenz und AF die Atemfrequenz ist.

4. Einrichtung nach einem der vorhergehenden Ansprüche, **dadurch gekennzeichnet, dass** der Quotient RAQ proportional zu oder gleich einem der folgenden Ausdrücke ist: $A_{rp}/AA_{vp}$, $A_{rp}/(AAvp/AF)$, $A_{rp}/(AA_{vp}*HF)$, $A_{rp}/(AA_{vp}*(HF/AF))$.

5. Einrichtung nach einem der vorangegangenen Ansprüche, **dadurch gekennzeichnet, dass** das charakteristische Signal die Laufzeit eines Ultraschallsignales ist, das aus dem Inneren des Schädels reflektiert wird.

6. Einrichtung nach einem oder mehreren der vorangegangenen Ansprüche, **dadurch gekennzeichnet, dass** die Einrichtung eine oder mehrere Einheiten aufweist, die dazu konfiguriert ist bzw. sind

a. die Pulsamplitude $A_{vp}$ jeder einzelnen Periode der modulierten Welle des charakteristischen Signals auszurechnen und ein Signal $A_{vp}$ oder eine Zeitreihe $A_{vp}$ zu erzeugen (3),
b. den mittleren Wert MICP für jede einzelne Periode der modulierten Welle des charakteristischen Signals zu berechnen und ein Signal MICP oder eine Zeitreihe MICP zu erzeugen (4),
c. die Amplitude $A_{rp}$ einer modulierenden Welle in der Zeitreihe MICP oder in dem Signal MICP zu berechnen (5),
d. die Amplitude $AA_{vp}$ der modulierenden Welle in der Zeitreihe $A_{vp}$ oder in dem Signal $A_{vp}$ zu berechnen (6),
e. die Kenngröße als Ausgangsgröße unter Verwendung zumindest einer oder mehrerer der Größen: $A_{vp}$, $A_{rp}$, $AA_{vp}$ zu berechnen.

7. Einrichtung nach Anspruch 6, **dadurch gekennzeichnet, dass** die jeweilige Einheit eine analoge Rechenschaltung, eine digitale Rechenschaltung oder eine programmgesteuerte Mikroprozessorschaltung ist.

8. Einrichtung nach einem der vorangegangenen Ansprüche, **dadurch gekennzeichnet, dass** die modulierte Welle eine der periodischen Signale aus der Gruppe der B-Wellen, C-Wellen, Atemwellen, herzschlagbedingte Wellen ist.

9. Einrichtung nach einem oder mehreren der vorangegangenen Ansprüche,
**dadurch gekennzeichnet, dass** die modulierende Welle eine der periodischen Signale aus der Gruppe der A-Wellen, B-Wellen, C-Wellen, Atemwellen ist, welche niederfrequenter als die modulierte Welle ist.

10. Einrichtung zur Bestimmung einer Kenngröße des Kopfinhaltes von Menschen und anderen Säugetieren, **gekennzeichnet durch** die Gewinnung und Auswertung eines für den Kopfinhalt charakteristischen Signales aufweisend eine ersten Einrichtung nach einem der vorangegangenen Ansprüche und eine zweite Einrichtung nach einem der vorangegangenen Ansprüche, wobei die erste Einrichtung dazu konfiguriert ist,

eine bestimmte Kombination von modulierter und modulierender Welle zu verwenden, und wobei die zweite Einrichtung dazu konfiguriert ist,
eine andere Kombination von modulierter und modulierender Welle zu verwenden, und wobei
die Einrichtung dazu konfiguriert ist, Kenngröße des Kopfinhaltes durch den Quotienten aus der Ausgangsgröße der ersten Einrichtung und der Ausgangsgröße der zweiten Einrichtung zu bilden.

11. Einrichtung nach einem der vorangegangenen Ansprüche, **dadurch gekennzeichnet, dass** die Einrichtung dazu konfiguriert ist, die Zeitreihe $A_{vp}$ zu bilden, indem für den Zeitabschnitt zwischen einem lokalen Minimum und dem darauf folgenden lokalen Maximum der modulierten Welle des charakteristischen Signals die Differenz zwischen dem lokalen Maximum und dem lokalen Minimum als Wert für die Zeitreihe $A_{vp}$ verwendet wird, und indem für den darauf folgenden Zeitabschnitt zwischen dem lokalen Maximum und dem darauf folgenden lokalen Minimum der modulierten Welle des charakteristischen Signals entsprechend die Differenz zwischen dem lokalen Maximum und dem darauf folgenden lokalen Minimum als Wert der Zeitreihe $A_{vp}$ verwendet wird, und/oder die Zeitreihe MICP zu bilden, indem für den Zeitabschnitt zwischen einem lokalen Minimum und dem darauf folgenden lokalen Maximum der modulierten Welle des charakteristischen Signals der mittlere Wert zwischen dem lokalen Maximum und dem lokalen Minimum als Wert für die Zeitreihe MICP verwendet wird, und indem für den darauf folgenden Zeitabschnitt zwischen dem lokalen Maximum und dem darauf folgenden lokalen Minimum der modulierten Welle des charakteristischen Signals entsprechend der mittlere Wert zwischen dem lokalen Maximum und dem darauf folgenden lokalen Minimum als Wert der Zeitreihe MICP verwendet wird.

12. Verfahren zur Bestimmung einer Kenngröße des Kopfinhaltes von Menschen und anderen Säugetieren unter Verwendung einer Einrichtung nach einem der vorhergehenden Ansprüche, aufweisend die Schritte:

   a. Ausrechnung der Pulsamplitude $A_{vp}$ jeder einzelnen Periode oder Halbperiode einer modulierten Welle des charakteristischen Signals und Erzeugung eines Signales $A_{vp}$ oder einer Zeitreihe $A_{vp}$ (3),
   b. Berechnung des mittleren Wertes MICP für jede einzelne Periode oder Halbperiode der modulierten Welle des charakteristischen Signals und Erzeugung eines Signales MICP oder einer Zeitreihe MICP (4),
   c. Berechnung der Amplitude $A_{rp}$ einer modulierenden Welle in der Zeitreihe MICP oder in dem Signal MICP (5),
   d. Berechnung der Amplitude $AA_{vp}$ der modulierenden Welle in der Zeitreihe $A_{vp}$ oder in dem Signal $A_{vp}$ (6),
   e. Berechnung der Kenngröße unter Verwendung zumindest einer oder mehrerer der Größen: $A_{vp}$, $A_{rp}$, $AA_{vp}$, wobei die Kenngröße ein Quotient ist, der die Größen $A_{rp}$ und $AA_{vp}$ aufweist.

## Claims

1. A device for determining a characteristic parameter of the head content of humans and other mammals with obtaining and processing of a signal characteristic of the head content as an input, wherein the characteristic signal is one of: the electrical impedance of the skull, the directly measured pressure inside the head, the electrical capacitance of the skull, the transit time of an ultrasonic signal passed through the skull, and wherein the device is configured to

   a. calculate the pulse amplitude $A_{vp}$ of each individual period or half period of a modulated wave of the characteristic signal and to generate a signal $A_{vp}$ or a time series $A_{vp}$ (3),
   b. calculate the mean value MICP for each single period or half period of the modulated wave of the characteristic signal and generate a signal MICP or a time series MICP (4),
   c. calculate the amplitude $A_{rp}$ of a modulating wave in the time series MICP or in the signal MICP (5),
   d. calculate the amplitude $AA_{vp}$ of the modulating wave in the time series $A_{vp}$ or in the signal $A_{vp}$ (6),
   e. calculate the characteristic parameter as an output value using at least one or more of the quantities: $A_{vp}$, $A_{rp}$, $AA_{vp}$, wherein the characteristic parameter is a quotient comprising the quantities $A_{rp}$ and $AA_{vp}$.

2. Device according to claim 1, **characterized in that** the characteristic parameter is a dimensionless parameter.

3. Device according to one of the preceding claims, **characterized in that** the characteristic parameter is a quotient comprising the quantities HF and / or AF, where HF is the heart rate and AF is the respiratory rate.

4. Device according to one of the preceding claims, **characterized in that** the quotient (RAQ) is proportional to or equal to one of the following expressions:

$$A_{rp}/AA_{vp}, \ A_{rp}/(AA_{vp}/AF), \ A_{rp}/(AA_{vp}*HF), \ A_{rp}/(AA_{vp}*(HF/AF)).$$

5. Device according to one of the preceding claims, **characterized in that** the characteristic signal is the transit time of an ultrasonic signal which is reflected from the interior of the skull.

6. Device according to one or more of the preceding claims, **characterized in that** the device comprises one or more units that is /are configured to

   a. calculate the pulse amplitude $A_{vp}$ of each individual period of the modulated wave of the characteristic signal and to generate a signal $A_{vp}$ or a time series $A_{vp}$ (3),
   b. calculate the mean value MICP for each individual period of the modulated wave of the characteristic signal and generate a signal MICP or a time series MICP (4),
   c. calculate the amplitude $A_{rp}$ of a modulating wave in the time series MICP or in the signal MICP (5),
   d. calculate the amplitude $AA_{vp}$ of the modulating wave in the time series $A_{vp}$ or in the signal $A_{vp}$ (6),
   e. calculate the characteristic parameter as an output value using at least one or more of the quantities: $A_{vp}$, $A_{rp}$,

$AA_{vp}$.

7. Device according to claim 6, **characterized in that** the respective unit is an analog computing circuit, a digital arithmetic circuit or a program-controlled microprocessor circuit.

8. Device according to one of the preceding claims, **characterized in that** the modulated wave is one of the periodic waves from the group of B-waves, C-waves, respiratory waves, heartbeat-induced waves.

9. Device according to one or more of the preceding claims, **characterized in that** the modulating wave is one of the periodic waves from the group of A-waves, B-waves, C-waves, respiratory waves, which has a lower frequency than the modulated wave.

10. Device for determining a characteristic parameter of the head content of humans and other mammals **characterized by** the extraction and evaluation of a head content characteristic signal, comprising a first device according to one of the preceding claims and a second device according to one of the preceding claims, wherein the first device is configured to use a particular combination of a modulated and a modulating wave, and wherein the second device is configured to use another combination of a modulated and a modulating wave, and wherein the device is configured to form the characteristic parameter of the head content as the quotient of the output value of the first device and the output value of the second device.

11. Device according to one of the preceding claims, **characterized in that** the device is configured to form the time series $A_{vp}$ by using for the time period from a local minimum to a subsequent local maximum of the modulated wave of the characteristic signal the difference between the local maximum and the local minimum as the value for the time series $A_{vp}$, and by using for the ensuing time period between the local maximum and the subsequent local minimum of the modulated wave of the characteristic signal the difference between the local maximum and the subsequent local minimum as the value of the time series $A_{vp}$, and / or to form the time series MICP by using for the time period between a local minimum and the subsequent local maximum of the modulated wave of the characteristic signal the average value between the local maximum and the local minimum as the value for the time series MICP, and by using for the subsequent time period between the local maximum and the subsequent local minimum of the modulated wave of the characteristic signal the average value between the local maximum and the subsequent local minimum as the value of time series MICP.

12. A method for determining a characteristic parameter of the head content of humans and other mammals using a device according to one of the preceding claims, comprising the steps:

   a. calculating the pulse amplitude $A_{vp}$ of each individual period or half period of a modulated wave of the characteristic signal and generating a signal $A_{vp}$ or a time series $A_{vp}$ (3),
   b. calculating the average value MICP for each individual period or half period of the modulated wave of the characteristic signal and generating a signal MICP or a time series MICP (4),
   c. calculating the amplitude $A_{rp}$ of a modulating wave in the time series MICP or in the signal MICP (5),
   d. calculating the amplitude $AA_{vp}$ of the modulating wave in the time series $A_{vp}$ or in the signal $A_{vp}$ (6),
   e. calculating the characteristic parameter using at least one or more of the following variables: $A_{vp}$, $A_{rp}$, $AA_{vp}$, wherein the characteristic parameter is a quotient comprising the quantities $A_{rp}$ and $AA_{vp}$.

**Revendications**

1. Dispositif pour la détermination d'une grandeur caractéristique du contenu cérébral d'êtres humains et autres mammifères, avec une obtention et évaluation d'un signal caractéristique du contenu cérébral en tant que grandeur d'entrée, dans lequel le signal caractéristique est un des signaux suivants : l'impédance électrique du crâne, la pression mesurée directement à l'intérieur de la tête, la capacité électrique du crâne, le temps de propagation d'un signal ultrasonore dirigé à travers le crâne, et dans lequel le dispositif est configuré pour

   a. calculer l'amplitude d'impulsion $A_{vp}$ de chaque période individuelle ou demi-période d'une onde modulée du signal caractéristique et générer un signal $A_{vp}$ ou une série chronologique $A_{vp}$ (3),
   b. calculer la valeur moyenne MICP pour chaque période individuelle ou demi-période de l'onde modulée du signal caractéristique et générer un signal MICP ou une série chronologique MICP (4),
   c. calculer l'amplitude $A_{rp}$ d'une onde modulante dans la série chronologique MICP ou dans le signal MICP (5),

d. calculer l'amplitude $AA_{vp}$ de l'onde modulante dans la série chronologique $A_{vp}$ ou dans le signal $A_{vp}$ (6),
e. calculer la grandeur caractéristique en tant que grandeur de sortie en utilisant au moins une ou plusieurs des grandeurs : $A_{vp}$, $A_{rp}$, $AA_{vp}$, dans lequel la grandeur caractéristique est un quotient qui présente les grandeurs $A_{rp}$ et $AA_{vp}$.

**2.** Dispositif selon la revendication 1, **caractérisé en ce que** la grandeur caractéristique est une grandeur caractéristique sans dimension.

**3.** Dispositif selon une des revendications précédentes, **caractérisé en ce que** la grandeur caractéristique est un quotient qui présente la grandeur HF et/ou AF, dans lequel HF est la fréquence cardiaque et AF est la fréquence respiratoire.

**4.** Dispositif selon une des revendications précédentes, **caractérisé en ce que** le quotient RAQ est proportionnel ou égal à une des expressions suivantes : $A_{rp}/AA_{vp}$,

$$A_{rp}/(AAvp/AF), \; A_{rp}/(AA_{vp}*HF), \; A_{rp}/(AA_{vp}*(HF/AF)).$$

**5.** Dispositif selon une des revendications précédentes, **caractérisé en ce que** le signal caractéristique est le temps de propagation d'un signal ultrasonore qui est réfléchi de l'intérieur du crâne.

**6.** Dispositif selon une ou plusieurs des revendications précédentes, **caractérisé en ce que** le dispositif présente un ou plusieurs modules qui est ou sont configurés pour

a. calculer l'amplitude d'impulsion $A_{vp}$ de chaque période individuelle de l'onde modulée du signal caractéristique et générer un signal $A_{vp}$ ou une série chronologique $A_{vp}$ (3),
b. calculer la valeur moyenne MICP pour chaque période individuelle de l'onde modulée du signal caractéristique et générer un signal MICP ou une série chronologique MICP (4),
c. calculer l'amplitude $A_{rp}$ d'une onde modulante dans la série chronologique MICP ou dans le signal MICP (5),
d. calculer l'amplitude $AA_{vp}$ de l'onde modulante dans la série chronologique $A_{vp}$ ou dans le signal $A_{vp}$ (6),
e. calculer la grandeur caractéristique en tant que grandeur de sortie en utilisant au moins une ou plusieurs des grandeurs : $A_{vp}$, $A_{rp}$, $AA_{vp}$.

**7.** Dispositif selon la revendication 6, **caractérisé en ce que** le module respectif est un circuit de calcul analogique, un circuit de calcul numérique ou un circuit de microprocesseur commandé par programme.

**8.** Dispositif selon une des revendications précédentes, **caractérisé en ce que** l'onde modulée est un des signaux périodiques provenant du groupe des ondes B, ondes C, ondes respiratoires, ondes conditionnées par le battement cardiaque.

**9.** Dispositif selon une ou plusieurs des revendications précédentes, **caractérisé en ce que** l'onde modulante est un des signaux périodiques provenant du groupe des ondes A, ondes B, ondes C, ondes respiratoires qui est de fréquence plus basse que l'onde modulée.

**10.** Dispositif pour la détermination d'une grandeur caractéristique du contenu cérébral d'êtres humains et autres mammifères, **caractérisé par** l'obtention et l'évaluation d'un signal caractéristique du contenu cérébral présentant un premier dispositif selon une des revendications précédentes et un second dispositif selon une des revendications précédentes, dans lequel le premier dispositif est configuré pour utiliser une combinaison déterminée d'onde modulée et modulante, et dans lequel le second dispositif est configuré pour utiliser une autre combinaison d'onde modulée et modulante, et dans lequel le dispositif est configuré pour former la grandeur caractéristique du contenu cérébral par le quotient provenant de la grandeur de sortie du premier dispositif et de la grandeur de sortie du second dispositif.

**11.** Dispositif selon une des revendications précédentes, **caractérisé en ce que** le dispositif est configuré pour former la série chronologique $A_{vp}$ **en ce que** la différence entre un maximum local et un minimum local est utilisée en tant que valeur pour la série chronologique $A_{vp}$ pour la période entre le minimum local et le maximum local subséquent de l'onde modulée du signal caractéristique, et **en ce que** la différence entre le maximum local et le minimum local subséquent est utilisée en tant que valeur de la série chronologique $A_{vp}$ pour la période subséquente entre le maximum local et le minimum local subséquent de l'onde modulée du signal caractéristique, et/ou pour former la série chronologique MICP **en ce que** la valeur moyenne entre un maximum local et un minimum local est utilisée en tant que valeur pour la série chronologique MICP pour la période entre le minimum local et le maximum local subséquent de l'onde modulée du signal caractéristique, et **en ce que** la valeur moyenne entre le maximum local et le minimum local subséquent est utilisée en tant que valeur de la série chronologique MICP pour la période subséquente entre le maximum local et le minimum local subséquent de l'onde modulée du signal caractéristique.

12. Procédé pour la détermination d'une grandeur caractéristique du contenu cérébral d'êtres humains et autres mammifères en utilisant un dispositif selon une des revendications précédentes, présentant les étapes de :

   a. calcul de l'amplitude d'impulsion $A_{vp}$ de chaque période individuelle ou demi-période d'une onde modulée du signal caractéristique et génération d'un signal $A_{vp}$ ou d'une série chronologique $A_{vp}$ (3),
   b. calcul de la valeur moyenne MICP pour chaque période individuelle ou demi-période de l'onde modulée du signal caractéristique et génération d'un signal MICP ou d'une série chronologique MICP (4),
   c. calcul de l'amplitude $A_{rp}$ d'une onde modulante dans la série chronologique MICP ou dans le signal MICP (5),
   d. calcul de l'amplitude $AA_{vp}$ de l'onde modulante dans la série chronologique $A_{vp}$ ou dans le signal $A_{vp}$ (6),
   e. calcul de la grandeur caractéristique en utilisant au moins une ou plusieurs des grandeurs : $A_{vp}$, $A_{rp}$, $AA_{vp}$, dans lequel la grandeur caractéristique est un quotient qui présente les grandeurs $A_{rp}$ et $AA_{vp}$.

Fig. 1

Fig. 2

Fig. 3A

Fig. 3B

Fig. 3C

Fig. 3D

Fig. 3E

Fig. 3F

Fig. 3G

Fig. 3H

**Fig. 3I**

**Fig. 3J**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 000002206624
- US 4787396 A
- DE 000019606687 A1
- US 673773407 B
- WO 20141167418 A

- US 20140371545 A1
- US 20050015009 A1
- US 6387051 B1
- US 2006079773 A1
- US 2013109979 A1

**In der Beschreibung aufgeführte Nicht-Patentliteratur**

- **S. JETZKI** ; **M. KIEFER** ; **R. EYMANN** ; **M. WALTER** ; **S. LEONHARDT**. Analysis of pulse waves in intracranial pressure. *Conference proceedings: Annual International Conference of the IEEE Engineering in Medicine and Biology Society. IEEE Engineering in Medicine and Biology Society. Conference*, 2006, vol. 2007, 2863-2866
- Attempts to continuously monitor autoregulation and compensatory reserve in severe head injuries. **D. J. PRICE** ; **M. CZOSNYKA** ; **M. WILLIAMSON**. Intracranial Pressure VIII. Springer, 1993, 61-66

- **M. CZOSNYKA** ; **Z. CZOSNYKA** ; **N. KEONG** ; **A. LAVINIO** ; **P. SMIELEWSKI** ; **S. MOMJIAN** ; **E. A. SCHMIDT** ; **G. PETRELLA** ; **B. OWLER** ; **J. D. PICKARD**. Pulse pressure waveform in hydrocephalus: what it is and what it isn't. *Neurosurg. Focus*, 2007, vol. 22 (4), 1-7
- **N. LENFELDT** ; **N. ANDERSSON** ; **A. ÅGREN-WILSSON** ; **A. T. BERGENHEIM** ; **L.-O. D. KOSKINEN** ; **A. EKLUND** ; **J. MALM**. Cerebrospinal fluid pulse pressure method: a possible substitute for the examination of B waves. *J. Neurosurg.*, 2004, vol. 101 (6), 944-950